# EUROPEAN PATENT SPECIFICATION

(11) **EP 2 077 991 B1**
(45) Date of publication and mention of the grant of the patent: **26.02.2014**
(21) Application number: 06809490.3
(22) Date of filing: 03.10.2006
(51) Int. Cl.: C07C 215/40, C07C 215/42, C07C 229/42, C07C 233/47, C07F 9/24

(54) **POSITIVELY CHARGED WATER-SOLUBLE PRODRUGS OF MUSTARDS AND RELATED COMPOUNDS WITH VERY HIGH SKIN PENETRATION RATES**
POSITIV GELADENE, WASSERLÖSLICHE PRODRUGS VON LOSTEN UND VERWANDTEN VERBINDUNGEN MIT SEHR HOHEN HAUTPENETRATIONSRATEN
PROMÉDICAMENTS DE MOUTARDES HYDROSOLUBLES À CHARGE POSITIVE ET COMPOSÉS APPARENTÉS PRÉSENTANT DES TAUX DE PÉNÉTRATION CUTANÉE TRÈS ÉLEVÉS

(43) Date of publication of application: 15.07.2009
(73) Proprietor: Techfields Biochem Co. Ltd, Shanghai N/A 200444 (CN); Yu, Chongxi, Plainfield, Illinois 60585 (US)
(72) Inventor: YU, Chongxi, Plainfield IL 60585 (US); XU, Lina, N/A 200444 Shanghai (CN)
(74) Representative: Viering, Jentschura & Partner Patent- und Rechtsanwälte
(86) International application number: PCT/IB2006/053619
(87) International publication number: WO 2008/041059

(56) References cited:
- WO-A1-01/00621
- WO-A1-96/20011
- US-A- 6 137 003
- US-A1- 2002 019 343
- DATABASE CAPLUS [Online] KLIUKIENE R., KARPAVICIUS K., KIL'DISHEVA O.V.: 'Synthesis of N-p-[bis(2-chloroethyl)amino]-phenacetylval ylmethionine diastereomers', XP008110611 Retrieved from STN Database accession no. (1977:55691) & IZVESTIYA AKADEMII NAUK SSSR, SERIYA KHIMICHESKAYA no. 9, 1976, pages 2081 - 2084
- DATABASE CAPLUS [Online] TIMAKOV V.D. ET AL.: 'Antiphagic and antibacterial activity of antitumor preparations. Dichloroethylamine and its derivatives', XP008110614 Retrieved from STN Database accession no. (1964:84692) & VOPROSY VIRUSOLOGII vol. 8, no. 6, 1963, pages 650 - 662
- DATABASE CAPLUS [Online] WOLF M. ET AL.: 'Alkylating benzamides with melanoma cytotoxicity', XP008110612 Retrieved from STN Database accession no. (2004:794497) & MELANOMA RESEARCH vol. 14, no. 5, 2004, pages 353 - 360

## Description

### Technical Field

The present invention relates to the preparations of positively charged and water-soluble pro-drugs of mustards and related compounds and their medicinal use in treating any mustards and related compounds-treatable conditions in humans or animals. More specifically, the present invention is to enable fast skin penetration of mustards and related compounds for reducing the side effects of these drugs in the treatment of cancers, psoriasis and other diseases.

### Background Art

Cancer is the second leading cause of death in the United States, accounting for almost 25% of deaths. Cancer chemotherapy has been under intensive development for the past 40 years, resulting in cures of certain types of disseminated cancers that previously were fatal.

There are very few differences between cancer and normal cells according to present knowledge. Almost every cancer drug destroys both cancer and normal cells, especially the rapidly dividing normal body cells, such as hair follicles, cells lining the gastrointestinal tract, and bone marrow cells involved in the immune defense system. The most common side effects of present chemotherapy are nausea, hair loss, and increased susceptibility to infection. In addition, there are many other side effects that cancer patients experience.

Mustards are alkylating agents. They are very reactive compounds that can react with DNA, RNA, and enzymes and then kill the cancer cells. Mustards are used for treatment of leukemias, breast, ovarian, and lung cancer. Springer, et al. designed and synthesized many nitrogen mustard compounds for the treatment of cancers (Springer, C.J. et al. U.S. Pat. No. 6852755, U.S. Pat. No.6916949, and U.S. Pat. No.6005002). Denny, et al. discussed the synthesis of nitrobenzyl mustard quaternary salts and their use as hypoxia-selective cytotoxic agents (Denny, W.A. et al. U.S. Pat. No.5691371). Glazier described prodrugs of phosphoramide mustard, isophosphoramide mustard and analogs (Glazier, A. U.S. Pat. No.5659061). Farquhar designed and synthesized novel antitumor aldophosphamide analogs (Glazier, A. U.S. Pat. No.5091552).

US patent application number US6137003 discloses bis (haloethyl) aminobenzene derivatives having carboxyl-containing moiety and another moiety at the ortho position with respect to the bis (haloethyl) amino substituent of the benzene ring.

### Disclosure of Invention

### Technical Problem

There are very few differences between cancer and normal cells according to present knowledge. Almost every cancer drug destroys both cancer and normal cells, especially the rapidly dividing normal body cells, such as hair follicles, cells lining the gastrointestinal tract, and bone marrow cells involved in the immune defense system. The most common side effects of present chemotherapy are nausea, hair loss, and increased susceptibility to infection. There are also many other side effects that cancer patients experience.

### Technical Solution

Kadow et al. described a novel method for the delivery of antitumor drugs to tumor cells by the administration of a tumor-selective antibody-beta-lactamase conjugate that binds to tumor cells (Kadow, J. et al. U.S. Pat. No. 5773435). One alternative method of administering cancer drugs is topical delivery. Topical cancer drug delivery has several advantages. This method helps to avoid directly hurting the gastro-intestinal tract by cancer drugs and inactivation of the drugs caused by first pass metabolism in the liver and gastro-intestinal tract. It can provide local delivery of appropriate concentrations of a drug to the intended site of action without systemic exposure. Topical drug delivery methods may use a much smaller amount of drugs than the amount used for the systemic method and thus reduce the side effects of cancer drugs.

This invention relates to the preparation of novel positively charged water-soluble pro-drugs of mustards and related compounds and their use medicinally according to the appended claims. Disclosed herein are pro-drugs of mustards and related compounds that have the general formula (1) 'Structure 1' or general formula (2) 'Structure 2'. 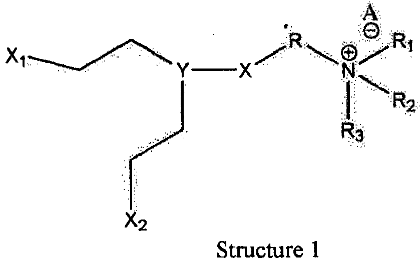 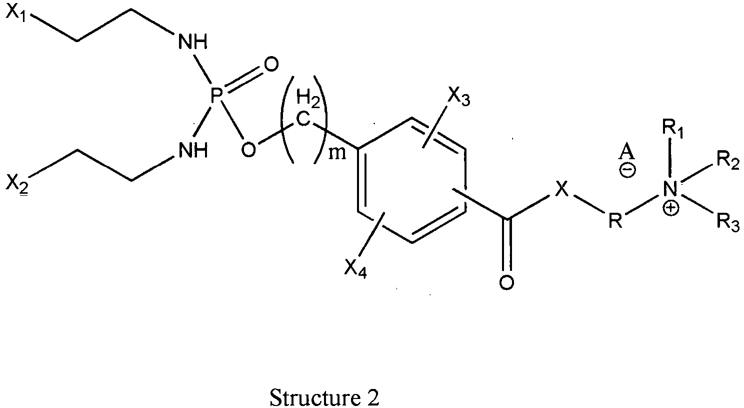 Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; A⁻ represents Cl⁻, Br⁻, F⁻, I⁻, AcO⁻, citrate, or any negative ions; R represents a branched or straight chain, - (CH₂)ₙ-, wherein n=0, 1, 2, 3, 4, 5, 6, 7, 8, 9, 10 ......; X represents O, S, NH; X₁ represents Cl, Br, F, I, OSO₂R₄(R₄ is lower alkyl, aryl, or heteroaryl groups), X₂ represents Cl, Br, F, I, OSO₂R₄(R₄ is lower alkyl, aryl, or heteroaryl groups). X₃ and X ₄ represent H, F, Cl, Br, I, NO₂, CN, CF₃, NHCOCH₃, OCH₃, SCH₃, NH_{2,} NHCH_{3,} OCOCH₃, OCOC₂H_{5,} OC₂H_{5,} OC₃H_{7,} CH₃,CH₂CH₅, C₃H₇; m=0, 1, 2, 3, 4, 5, 6, 7, 8, ......; All R, -(CH₂)ₙ- or -(CH₂)ₘ-groups are branched or straight chains and may include C, H, O, S, or N atoms and may have single, double, and triple bonds. Any CH₂ groups may be replaced with O, S, or NH. Y represents: 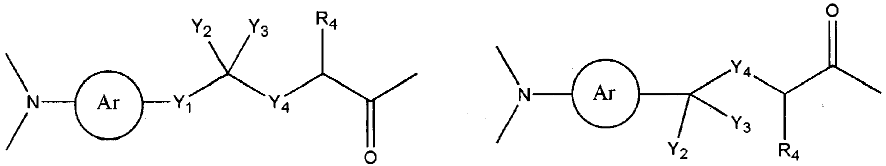 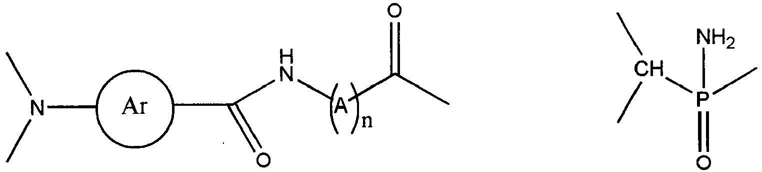 wherein Y₁ represents CH₂, O, S, NH; Y₂ and Y₃ taken alone are different and are H, OH, NHCOCH₃, NHCOC₂H₅, Cl, F, Br, I, or taken together are oxygen or 2 hydrogens; Y₄ represents CH₂, -(CH₂)ₙ-, O, S, NH; R₄ represents H, CH₂CONH₂, CH₂ CH₂CONH₂, CH₂COOCH₃, CH₂COOC₂H₅, CH₂NHCOCH₃, CH₂CH₂NHCOCH₃, CH₂ CH₂CH₂NHCOCH₃, CH₂CH₂CH₂CH₂NHCOCH₃, one of any alkyl, alkyloxyl, alkenyl or alkynyl residues having 1 to 12 carbon atoms, aryl or heteroaryl residues, A represents α-amino acid or β-amino acid residues, n=0, 1, 2, 3, 4, 5, 6, 7......; All R, - (CH₂)ₙ- or -(CH₂)ₘ-groups are branched or straight chains and may include C, H, O, S, or N atoms and may have single, double, and triple bonds. Any CH₂ groups may be replaced with O, S, or NH. 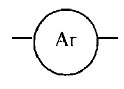 represents one of any ary or heteroaryl systems, they include, but are not limited to: 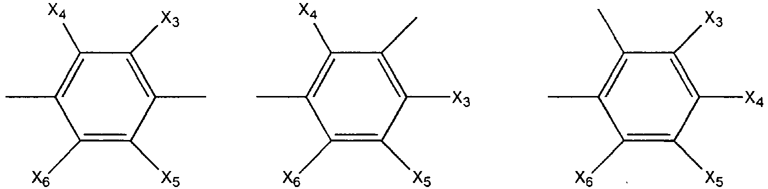 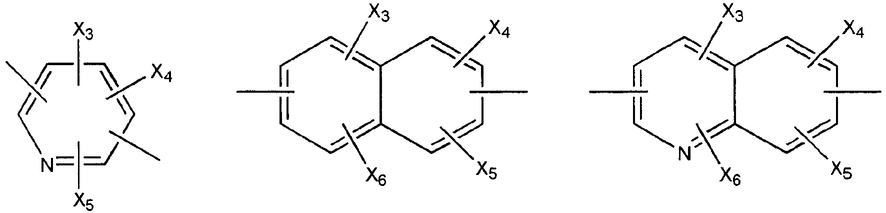 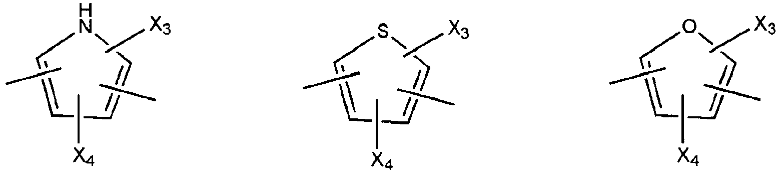 Wherein, X₃, X_{4,}X_{5,} and X₆ represent H, F, Cl, Br, I, NHCOCH₃, NO₂, CN, CF₃, OCH₃ , SCH_{3,} NH_{2,} NHCH_{3,} OCH₃, OC₂H_{5,} OC₃H_{7,} CH₃, C₂H₅, C₃H_{7.} When the bonds are not linked with atoms of the aryl or heteroaryl ring, that means that the bonds can be put into any position of the ring.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

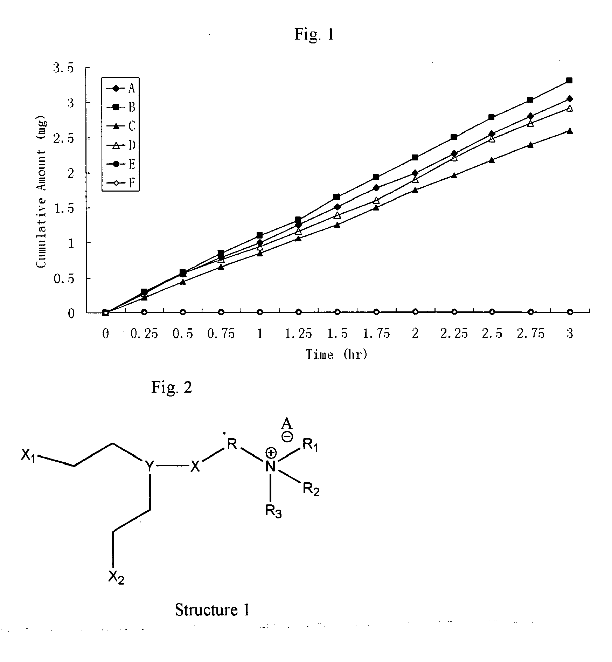

The goal of this invention is to make mustards and related compounds administrable transdermally (topical application) by increasing their solubility in the moisture on the skin surface and their penetration rate through the membrane and skin barrier to avoid hurting the GI tract. These novel pro-drugs of mustards and related compounds have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water. The solubility of theses pro-drugs of mustards and related compounds is >250 mg/ml and the solubility of mustards and related compounds is <0.1 mg/ml. In many instances, the lowest or rate-limiting step in the sequence is the dissolution of the drug. Mustards and related compounds have a very low solubility in the moisture on the skin surface and they will not pass across the barrier of skin in a molecular form efficiently. They stay outside of skin membranes for a long time and thus, will hurt the skin. When these new pro-drugs are administered transdermally in a dosage form such as a solution, spray, lotion, ointment, emulsion or gel, they will dissolve in the moisture of the skin surface immediately. The positive charge on the amino groups of these pro-drugs will bond to the negative charge on the phosphate head group of the membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay outside of skin membranes and thus, the pro-drugs will not hurt the skin. The penetration rates of these prodrugs through human skin were measured in vitro by using modified Franz cells, which were isolated from human skin tissue (360-400 µm thick) of the anterior and posterior thigh areas. The receiving fluid consisted of 2 ml of 2% pH 7.4 phosphate buffer (0.2 M) and was stirred at 600 rpm. The cumulative amounts of these prodrugs and their parent drugs penetrating the skin versus time were determined by a specific high-performance liquid chromatography method. The results using a donor consisting of either a 20% solution of some of the prodrugs or a 20% suspension of some of the mustards and related compounds in 0.2mL of pH 7.4-phosphate buffer (0.2M) are shown in Figure 1. Apparent flux values of 1.01 mg, 1.10 mg, 0.85 mg, 0.94 mg, 0.01 mg, and 0.01 mg/cm²/h were calculated for N,N-diethylaminoethyl 4-[bis(2-chloroethyl)amino]benzenebutyrate.HBr, 4-[bis(2-chloroethyl)amino] - N-acetyl-L-phenylalanine N,N-diethylaminoethyl ester hydrobromide, N,N-bis(2-chloroethyl)aminophosphamide N,N-diethylaminoethyl ester hydrobromide, diethylaminoethyl 4-[bis(2-methylsulfonylethyl)amino]benzenebutyrate.HBr, chlorambucil, and melphalan diffuse through human skin. The results suggest that the pro-drugs diffuse through human skin ∼ 100 times faster than do mustards and related compounds. The results suggest that the positive charge on the dialkyaminoethyl group has a very important role in the passage of the drug across the membrane and skin barrier. Other prodrugs of the general formula (1) 'Structure 1a' and general formula (2) 'Structure 2a' according to the appended claims have very high penetration rates and are very close to that of N,N-diethylaminoethyl 4-[bis(2-chloroethyl)amino]benzenebutyrate.HBr.

For evaluation of antitumor activity, a human myeloma cell line derived from the ascites of a patient with multiple myeloma was implanted into mice. The experiment was carried out on 11 groups of mice. Control group (A, orally), melphalan (B₁ and B₂, orally), chlorambucil (C₁ and C₂, orally), N,N-diethylaminoethyl 4-[bis(2-chloroethyl)amino]benzenebutyrate.HBr (D₁ and D₂, transdermally), 4-[bis(2-chloroethyl)amino]-N-acetyl-L-phenylalanine N,N-diethylaminoethyl ester hydrobromide (E and E₂, transdermally), and diethylaminoethyl 4-[bis(2-methylsulfonylethyl)amino]benzenebutyrate.HCl (F₁ and F₂, transdermally). The body weight loss of mice was determined on day 21. The results are shown in table 1.

**Table 1: Extension of survival period and weight loss of cancer mice by use of mustards and their novel prodrugs.**

| Compounds | Dose (mg/kg) perday | Numbers of Mice | Survival Period (days) | Life Elongation Rate(%) | None Disease Rate | Weight Loss (%) |
|---|---|---|---|---|---|---|
| Control (A) | - | 7 | 45.5±1.6 | 100 | 0/7 | 1% |
| B₁ | 1.5 | 7 | 55.7±1.3 | 122 | 0/7 | 10% |
| B₂ | 3.0 | 7 | 88.5±1.8 | 195 | 2/7 | 18% |
| C₁ | 1.5 | 7 | 57.8±1.5 | 127 | 0/7 | 9% |
| C₂ | 3.0 | 7 | 90.2±1.9 | 198 | 3/7 | 17% |
| D₁ | 1.5 | 7 | 128.5±1. 3 | 282 | 4/7 | 5% |
| D₂ | 3.0 | 7 | 115.2±2. 1 | 253 | 4/7 | 10% |
| E₁ | 1.5 | 7 | 130.5±1. 6 | 287 | 4/7 | 4% |
| E ₂ | 3.0 | 7 | 121.2±1. 8 | 266 | 3/7 | 9% |
| F ₁ | 1.5 | 7 | 122.5±1. 7 | 269 | 4/7 | 6% |
| F ₂ | 3.0 | 7 | 111.2±1. 9 | 244 | 3/7 | 11% |

The results show that the prodrugs demonstrated strong antitumor activity at 1.5 mg/kg dose and caused much less side effects (less weight loss) when they were administered transdermally.

All mustards that we used to make the prodrugs are known compounds. They are commercially available or can be made according to literature. The prodrugs of the general formula (1) 'Structure 1' and general formula (2) 'Structure 2 ' indicated above can be prepared from mustards and related compounds, by reaction with compounds of the general formula (3) 'Structure 3' by using coupling reagents, such as N,N'-Dicyclohexylcarbodiimide and N, N'-Diisopropylcarbodiimide, et al. 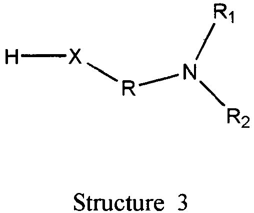 Wherein , R represents a branched or straight chain, -(CH₂)ₙ-, and n=0, 1, 2, 3, 4, 5, 6, 7, 8, 9, 10 .......; R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; X represents O, S or NH; and n=0, 1, 2, 3, 4, 5, 6, 7, 8, 9, 10......

When X represents O, the compounds of the general formula (1) 'Structure 1' and general formula (2) 'Structure 2' indicated above can be prepared from metal salts, organic base salts, or immobilized base salts of mustards and related compounds, by reaction with compounds of the general formula (4) 'Structure 4'. 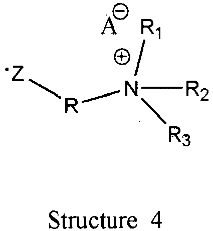 Wherein, R represents a branched or straight chain, -(CH₂)ₙ-, and n=0, 1, 2, 3, 4, 5, 6, 7, 8, 9, 10 ......; R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; Z represents halogen, or p-toluenesulphonyl, A⁻ represents Cl⁻, Br⁻, F, I⁻, AcO⁻, citrate, or any negative ions.

### Advantageous Effects

These pro-drugs of mustards and related compounds in the present invention have a lipophilic portion and a hydrophilic portion (the amine groups that exist in the protonated form at physiological pH). The positively charged amino groups of these pro-drugs have two major advantages. First, it largely increases the solubility of the drugs in water; when these new pro-drugs are administered transdermally in a dosage form such as a solution, spray, lotion, ointment, emulsion or gel , they will mix with moisture on the skin or other part of the body immediately. Second, the positive charge on the amino group of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay on the skin or other part of the body, the pro-drugs will hurt the skin or other part of the body much less. Experiment results show that more than 90% of the pro-drugs were changed back to the parent drugs in a few minutes. The pro-drugs have a much better absorption rate and transdermal administration avoids the first pass metabolism , therefore the pro-drugs will have more strength than will mustards and related compounds at the same dosage. Another great benefit of transdermal administration of these pro-drugs is that administering medication, especially to children, will be much easier.

### Description of Drawings

Figure 1: Cumulative amounts of N,N-diethylaminoethyl 4-[bis(2-chloroethyl)amino]benzenebutyrate.HBr (A, 20% solution), 4-[bis(2-chloroethyl)amino]-N-acetyl-L-phenylalanine N,N-diethylaminoethyl ester hydrobromide (B, 20% solution), N,N-bis(2-chloroethyl)aminophosphamide N,N-diethylaminoethyl ester hydrobromide (C, 20% solution), diethylaminoethyl 4-[bis(2-methylsulfonylethyl)amino]benzenebutyrate.HBr (D, 20% solution), chlorambucil (E, 20% suspension), and melphalan (F, 20% suspension) crossing isolated human skin tissue in Franz cells (n=5). In each case, the vehicle was pH 7.4 phosphate buffer (0.2 M).
Figure 2. Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; A⁻ represents Cl⁻, Br⁻, F, I⁻, AcO⁻, citrate, or any negative ions; R represents a branched or straight chain, - (CH₂)ₙ-, and n=0, 1, 2, 3, 4, 5, 6, 7, 8, 9, 10 ......; X represents O, S, NH, X₁ represents Cl, Br, F, I, OSO₂R₄(R₄ is lower alkyl, aryl, or heteroaryl groups), X₂ represents Cl, Br, F, I, OSO₂R₄(R₄ is lower alkyl, aryl, or heteroaryl groups). See the Claim 1 for Y definition.

### Best Mode

### Preparation of N,N-diethylaminoethyl 4-[bis(2-chloroethyl)amino] benzenebutyrate.HBr

32.6 g (0.1 mol) of sodium 4-[bis(2-chloroethyl)amino]benzenebutyrate was dissolved in 100 ml of acetonitrile. 26 g (0.10 mol) of 2-Bromo-N,N-diethylethylamine.HBr in 50 ml of acetonitrile was added into the reaction mixture. The mixture was stirred for 3 h at RT. Solid is removed by filtration. The solvents were evaporated off. The solid product was collected by filtration and washed with ether. After drying, it yielded 35 g of the desired product (72.3%). Hygroscopic product; Solubility in water: 300 mg/ml; Elementary analysis: C₂₀H₃₃BrCl₂N₂ O₂; MW: 484.30. Calculated % C: 49.60, H: 6.87, Br: 16.50, N: 5.78, O: 6.61 ; Cl: 14.64; Found % C: 49.52, H: 6.89, Br: 16.55 N: 5.75, O: 6.65; Cl: 14.64. ¹H-NMR (400 MHz, D₂O): δ: 1.55 (t, 6H), 2.02(m, 2H), 2.27 (m, 2H), 2.54 (m, 2H), 3.23 (m, 4H), 3.51 (m, 2H), 3.60-3.65 (m, 8H), 4.51 (m, 2H), 6.55 (m, 2H), 6.95 (m, 2H).

### Mode for Invention

### Preparation of N,N-diethylaminoethyl 4-[bis(2-chloroethyl)aminol benzenebutyrate.HCl

30.4 g (0.1 mol) of 4-[bis(2-chloroethyl)aminolbenzenebutanoic acid was dissolved in 300 ml of chloroform. 20.6 g of N, N'-Dicyclohexylcarbodiimide was added into the reaction mixture. 11.7 g of N,N-diethylaminoethanol and 0.2 g of 4-dimethylaminopyridine were added into the reaction mixture. The mixture was stirred overnight at 0°C. The solid was removed by filtration. The chloroform solution was washed with water (1 x 100 ml), 5% NaHCO₃ (1 x 100 ml) and water (3 x 100 ml). The organic solution was dried over anhydrous sodium sulfate. Sodium sulfate was removed by filtration. 4 g of HCl gas in methanol (10 ml) was added into the reaction mixture with stirring. Hexane (200 ml) was added. The solid product was collected by filtration. After drying, it yielded 35 g of the desired product (79.6 %). Hygroscopic product; Solubility in water: 300 mg/ml; Elementary analysis: C₂₀H₃₃Cl₃N ₂O₂; MW: 439.85. Calculated % C: 54.61, H: 7.56, N: 6.37; O: 7.27; Cl: 24.18; Found % C: 54.55; H: 7.58; N: 6.34, O: 7.29; Cl: 24.24. ¹H-NMR (400 MHz, D₂O): δ: 1.56 (t, 6H), 2.01 (m, 2H), 2.25 (m, 2H), 2.55 (m, 2H), 3.22 (m, 4H), 3.52 (m, 2H), 3.60-3.65 (m, 8H), 4.50 (m, 2H), 6.55 (m, 2H), 6.95 (m, 2H).

### Preparation of 4-[bis(2-chloroethyl)amino]-N-acetyl-L-phenylalanine N,N-diethylaminoethyl ester hydrobromide

36.9 g (0.1 mol) of sodium 4-[bis(2-chloroethyl)amino]-N-acetyl-L-phenylalanine was dissolved in 100 ml of acetonitrile. 26 g (0.10 mol) of 2-Bromo-N,N-diethylethylamine.HBr in 100 ml of acetonitrile was added into the reaction mixture. The mixture was stirred for 3 h at RT. Solid is removed by filtration. The solvents were evaporated off. The solid product was collected by filtration and washed with ether. After drying, it yielded 38 g of the desired product (72.1 %). Hygroscopic product; Solubility in water: 300 mg/ml; Elementary analysis: C₂₁H₃₄BrCl₂N₂ O₂; MW: 527.32. Calculated % C: 47.83, H: 6.50, Br: 1.5.15, N: 7.97, O: 9.10, Cl, 13.45; Found % C: 47.77, H: 6.52, Br: 15.12 N: 7.96, O: 9.15; Cl: 13.48. ¹H-NMR (400 MHz, D₂O): δ:1.54 (t, 6H), 2.02(s, 3H), 3.16 (m, 2H), 3.23 (m, 4H), 3.51 (m, 2H), 3.60-3.65 (m, 8H), 4.51 (m, 2H), 4.81 (m, 1H), 6.55 (m, 2H), 6.95 (m, 2H).

### Industrial Applicability

The pro-drugs of the general formula (1) 'Structure 1a' and general formula (2) 'Structure 2a' according to the appended claims are superior to mustards and related compounds. They can be used medicinally in treating any mustards and related compounds-treatable conditions in humans or animals. They may be used for the treatment of skin cancer, breast cancer, oral cancer, colon-rectum cancer, respiratory system cancer, genital cancer, leukemia, or other cancers. They may also be used for the treatment of psoriasis.

## Claims

1. A compound selected from the group consisting of N,N-bis(2-chloroethyl)aminophosphamide N,N-diethylaminoethyl ester hydrobromide, 4-[bis(2-chloroethyl)amino] - N-acetyl-L-phenylalanine N,N-diethylaminoethyl ester hydrobromide, diethylaminoethyl 4-[bis(2-methylsulfonylethyl)amino]benzenebutyrate.HBr and a compound having a general formula of Structure 1a 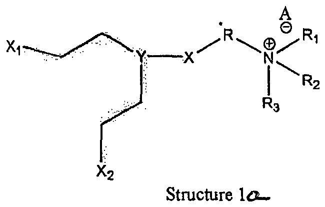
wherein R₁ is selected from the group consisting of H, alkyl, alkyloxy, alkenyl, and alkynyl residues having up to 12 carbon atoms, aryl, and heteroaryl residues;
R₂ is selected from the group consisting of H, alkyl, alkyloxy, alkenyl, and alkynyl residues having up to 12 carbon atoms, aryl, and heteroaryl residues;
R₃ is H;
A⁻ is selected from any negative ions;
R is selected from the group consisting of a branched or straight chain, -(CH₂)ₙ-, wherein n= 0, 1, 2, 3, 4, 5, 6, 7, 8, 9, or 10;
X is selected from the group consisting of O, S, and NH;
X₁ is selected from the group consisting of Cl, Br, F, I, and OSO₂R₄ (R₄ is aryl, or heteroaryl groups);
X₂ is selected from the group consisting of Cl, Br, F, I, and OSO₂R₄ (R₄ is aryl, or heteroaryl groups);
Y is selected from the group consisting of 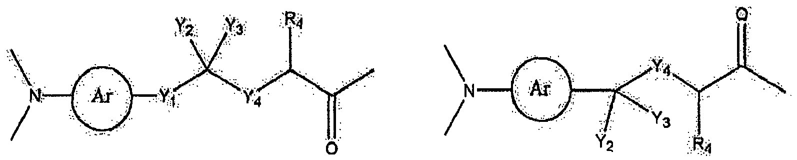 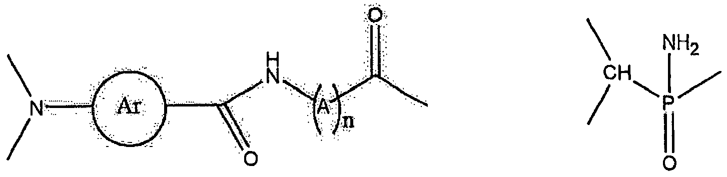
wherein Y₁ is selected from the group consisting of CH₂, O, S, and NH;
Y₂ and Y₃ are independently selected from the group consisting of H, OH, NHCOCH₃, NHCOC₂H₅, Cl, F, Br, and I, or taken together are oxygen or 2 hydrogens;
Y₄ is selected from the group consisting of CH₂, -(CH₂)ₚ-, wherein p=0, 1, 2, 3, 4, 5, 6, or 7, O, S, and NH;
R₄ is selected from the group consisting of H, CH₂CONH₂, CH₂CH₂CONH₂, CH₂COOCH₃, CH₂CNOOC₂H₅, CH₂NHCOCH₃, CH₂CH₂NHCOCH₃, CH₂CH₂CH₂NHCOCH₃,
CH₂CH₂CH₂CH₂NHCOCH₃, alkyl, alkyloxy, alkenyl, and alkynyl residues having to 12 carbon atoms, aryl, and heteroaryl residues;
A is selected from the group consisting of α-amino acid and β-amino acid residues; 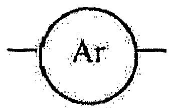 is selected from the group consisting of: 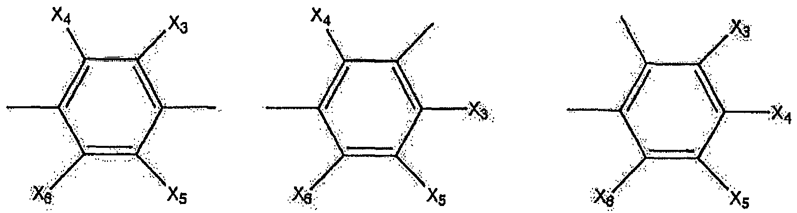 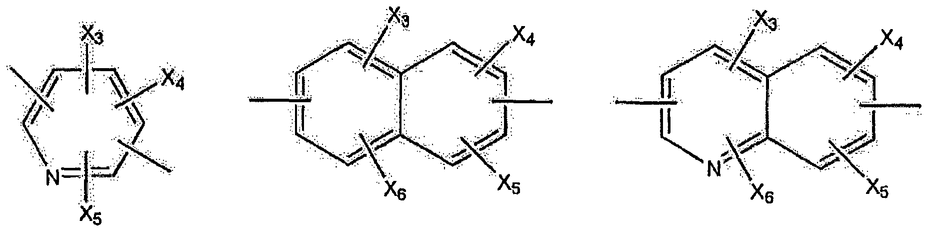 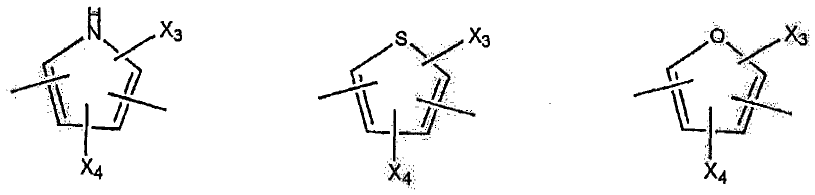 wherein X₃ , X₄, X₅, and X₆ are independently selected from the group consisting of H, F, Cl, Br, I, NHCOCH₃, NO₂ , CN, CF₃, OCH₃, SCH₃, NH₂, NHCH₃, OCH₃, OC₂H₅, OC₃H₇, CH₃, C₂H₅, and C₃H₇, wherein when the bonds are not linked with atoms of the aryl or heteroaryl ring, that means that the bonds can be put into any position of the ring.

2. A compound having a general formula of Structure 2a 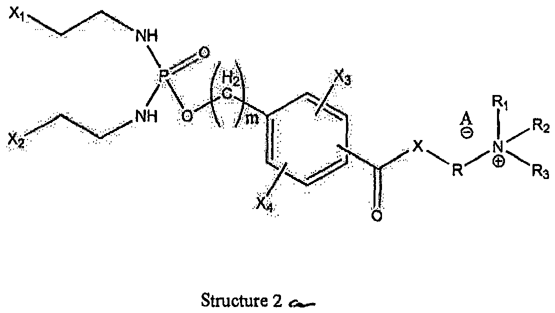
wherein R₁ is selected from the group consisting of H, alkyl, alkyloxy, alkenyl, and alkynyl residues having up to 12 carbon atoms, aryl, and heteroaryl residues;
R₂ is selected from the group consisting of H, alkyl, alkyloxy, alkenyl, and alkenyl residues having up to 12 carbon atoms, aryl, and heteroaryl residues;
R₃ is H;
A⁻ is selected from any negative ions;
R is selected from the group consisting of a branched or straight chain, -(CH ₂)ₙ-, wherein n=0, 1, 2, 3, 4, 5, 6, 7, 8, 9, or 10, aryl, and heteroaryl residues;
X is selected from the group consisting of O, S, and NH;
X₁ is selected from the group consisting of Cl, Br, F, I, and OSO₂R₄ (R₄ is aryl, or heteroaryl groups);
X₂ is selected from the group consisting of Cl, Br, F, I, and OSO₂R₄ (R₄ is aryl, or heteroaryl groups);
X₃ and X₄ are independently selected from the group consisting of H, F, Cl, Br, I, NO₂, CN, CF₃, NHCOCH₃, OCH₃, SCH₃, NH₂, NHCH₃, OCOCH₃, OCOC₂H₅, OC₂H₅, OC₃H₇, CH₃, C₂H₅, and C₃H₇; m=0, 1, 2,3,4,5,6, 7, or 8;.

3. Process for the preparation of a compound according to claim 1 or claim 2, wherein the compound are prepared from mustard compounds by reaction with compounds having a general formula of Structure 3a by use of a coupling reagent selected from the group consisting of N,N'-dicyclohexylcarbodiimide, N, N'-diisopropylcarbodiimide, O-(benzotriazol-1-yl)-N,N,N,N'-tetramethyluronium tetrafluoroborate, O- (benzotriazol-1-yl)-N,N,N',N'-tetramethyluroniumhexafluorophosphate, benzotriazol-1-yl-oxy-tris(dimethylamino)phosphoniumhexafluorophosphate 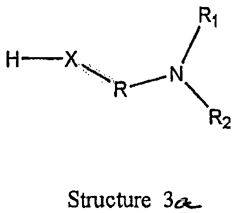
wherein R is selected from the group consisting of a branched or straight chain, -(CH₂)ₙ-, and n=0, 1, 2, 3, 4, 5, 6, 7, 8, 9, or 10;
R₁ is selected from the group consisting of H, alkyl, alkyloxy, alkenyl, and alkynyl residues having up to 12 carbon atoms, aryl, and heteroaryl residues;
R₂ is selected from the group consisting of H, alkyl, alkyloxy, alkenyl, and alkynyl residues having up to 12 carbon atoms, aryl, and heteroaryl residues;
X is selected from the group consisting of O, S, and NH.

4. Process for the preparation of a compound according to Claim 1 or Claim 2, wherein metal salts, organic base salts, or immobilized base salts of mustard compounds are reacted with compounds having a general formula of Structure 4a 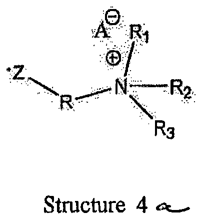
wherein R is selected from the group consisting of a branched or straight chain, -(CH₂)ₙ-, and n=0, 1, 2, 3, 4, 5, 6, 7, 8, 9, or 10;
R₁ is selected from the group consisting of H, alkyl, alkyloxy, alkenyl, and alkynyl residues having up to 12 carbon atoms, aryl, and heteroaryl residues;
R₂ is selected from the group consisting of H, alkyl, alkyloxy, alkenyl, and alkynyl residues having up to 12 carbon atoms, aryl, and heteroaryl residues;
R₃ is H;
Z is halogen or p-toluenesulphonyl;
A⁻ is selected from any negative ions.

5. The compound according to claims 1 and 2 or a composition comprising at least one compound according to claims 1 and 2, as an active ingredient, for use in a method of treatment in human or animal, wherein the treated condition is selected from the group consisting of skin cancer, breast cancer, oral cancer, colon-rectwn cancer, respiratory system cancer, genital cancer, leukemia, other cancers, and psoriasis, and the compound or composition is transdermally or orally administered.

6. Transdermal therapeutic application systems comprising a compound according to claim 1 or 2 or a composition comprising at least one compound according to claim 1 or claim 2, as an active ingredient, for use in a method of treatment in human or animal, wherein the treated condition is selected from the group consisting of skin cancer, breast cancer, oral cancer, colon-rectum cancer, respiratory system cancer, genital cancer, leukemia, other cancers, and psoriasis.

7. The transdermal therapeutic application systems for use according to claim 6, **characterized in that** these systems are a bandage or a patch comprising of one active substance-containing matrix layer and an impermeable backing layer.

8. The transdermal therapeutic application systems for use according to claims 6 or 7, **characterized by** having an active substance reservoir, which has a permeable bottom facing the skin.

9. The transdermal therapeutic application systems for use according to one of claims 6-8, **characterized by** a controlling means for controlling the rate of release, enabling this system to reach constantly optimal therapeutic blood levels of the mustard compounds to increase effectiveness and reduce the side effects of mustard compounds.

## Patentansprüche

1. Eine Verbindung, ausgewählt aus der Gruppe bestehend aus N,N-Bis(2-chlorethyl)-aminophosphamid-N,N-diethylaminoethylesterhydrobromid, 4-[Bis(2-chlorethyl)amino]-N-Acetyl-L-phenylalanin-N,N-diethylaminoethylesterhydrobromid, Diethylaminoethyl-4-[bis(2-methylsulfonylethyl)amino]benzolbutyrat.HBr und einer Verbindung, die eine allgemeine Formel der Struktur 1 a 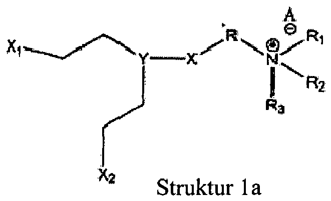 besitzt,
wobei R₁ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
R₂ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
R₃ H ist;
A⁻ ausgewählt ist aus beliebigen negativen Ionen;
R ausgewählt ist aus der Gruppe bestehend aus einem verzweigten oder geradkettigen -(CH₂)ₙ-, wobei n = 0, 1, 2, 3, 4, 5, 6; 7, 8, 9 oder 10 ist;
X ausgewählt ist aus der Gruppe bestehend aus O, S und NH;
X₁ ausgewählt ist aus der Gruppe bestehend aus Cl, Br, F, I und OSO₂R₄ (R₄ ist eine Aryl oder Heteroaryl Gruppe);
X₂ ausgewählt ist aus der Gruppe bestehend aus Cl, Br, F, I und OSO₂R₄ (R₄ ist eine Aryl oder Heteroaryl Gruppe);
Y ausgewählt ist aus der Gruppe bestehend aus 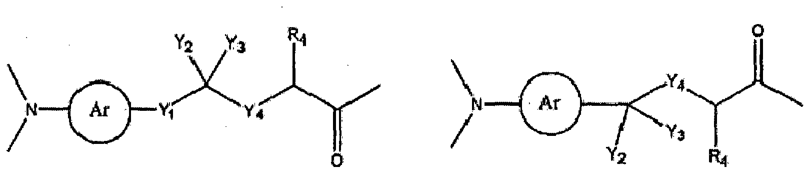 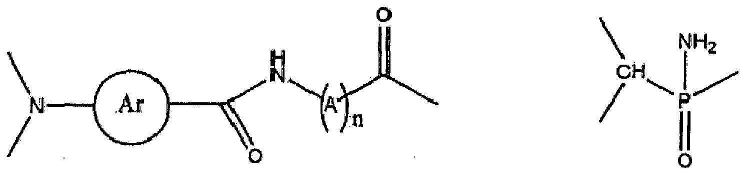
wobei Y₁ ausgewählt ist aus der Gruppe bestehend aus CH₂, O, S und NH;
Y₂ und Y₃ unabhängig ausgewählt sind aus der Gruppe bestehend aus H, OH, NHCOCH₃, NHCOC₂H₅, Cl, F, Br und I, oder zusammengenommen ein Sauerstoff- oder 2 Wasserstoffatome sind;
Y₄ ausgewählt ist aus der Gruppe bestehend aus CH₂, -(CH₂)ₚ-, wobei p = 0, 1, 2, 3, 4, 5, 6 oder 7, O, S und NH ist;
R₄ ausgewählt ist aus der Gruppe bestehend aus H, CH₂CONH₂, CH₂CH₂CONH₂, CH₂COOCH₃, CH₂CNOOC₂H₅, CH₂NHCOCH₃, CH₂CH₂NHCOCH₃, CH₂CH₂CH₂NHCOCH₃, CH₂CH₂CH₂CH₂NHCOCH₃, Alkyl, Alkyloxy, Alkenyl und Alkinyl Resten mit bis zu 12 Kohlenstoffatomen, Aryl und Heteroaryl Resten;
A ausgewählt ist aus der Gruppe bestehend aus α-Aminosäure- und β-Aminosäure-Resten; 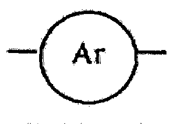 ausgewählt ist aus der Gruppe bestehend aus 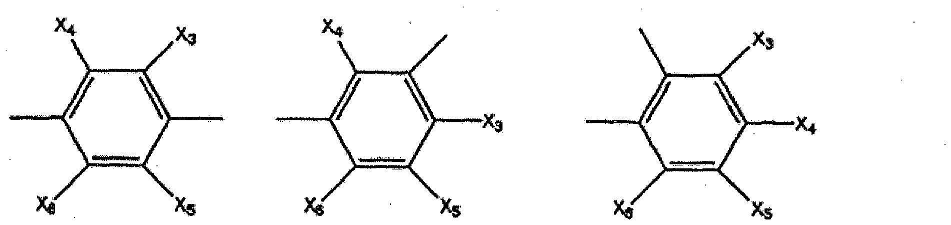 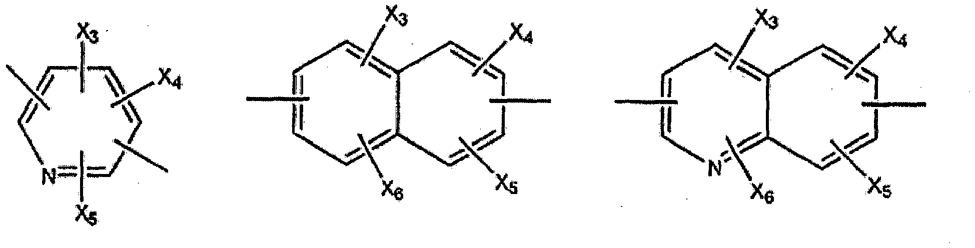 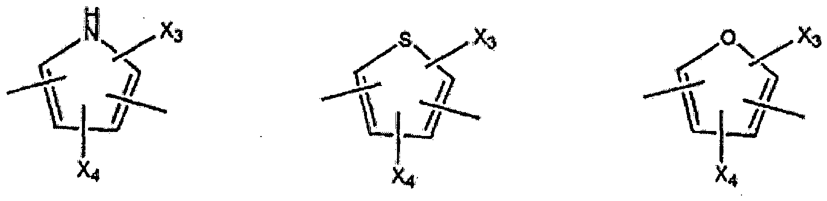 wobei X₃, X₄, X₅ und X₆ unabhängig ausgewählt sind aus der Gruppe bestehend aus H, F, Cl, Br, I, NHCOCH₃, NO₂, CN, CF₃, OCH₃, SCH₃, NH₂, NHCH₃, OCH₃, OC₂H₅, OC₃H₇, CH₃, C₂H₅ und C₃H₇, wobei, wenn die Bindungen nicht mit Atomen des Aryl- oder Heteroaryl-Rings verbunden sind, es bedeutet, dass die Bindungen an jede Position des Rings gestellt werden können.

2. Eine Verbindung, die eine allgemeine Formel der Struktur 2a 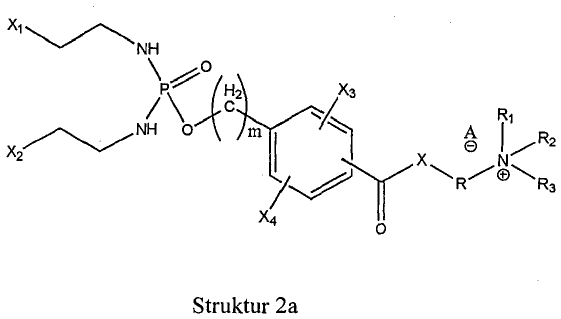 besitzt,
wobei R₁ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
R₂ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
R₃ H ist;
A⁻ ausgewählt ist aus beliebigen negativen Ionen;
R ausgewählt ist aus der Gruppe bestehend aus einem verzweigten oder geradkettigen -(CH₂)ₙ-, wobei n = 0 , 1, 2 , 3, 4 , 5, 6 , 7, 8 , 9 oder 10 ist, Aryl und Heteroaryl Resten;
X ausgewählt ist aus der Gruppe bestehend aus O, S und NH;
X₁ ausgewählt ist aus der Gruppe bestehend aus Cl, Br, F, I und OSO₂R₄ (R₄ ist eine Aryl oder Heteroaryl Gruppe);
X₂ ausgewählt ist aus der Gruppe bestehend aus Cl, Br, F, I und OSO₂R₄ (R₄ ist eine Aryl oder Heteroaryl Gruppe);
X₃ und X₄ unabhängig ausgewählt sind aus der Gruppe bestehend aus H, F, Cl, Br, I, NO₂, CN, CF₃, NHCOCH₃, OCH₃, SCH₃, NH₂, NHCH₃, OCOCH₃, OCOC₂H₅, OC₂H₅, OC₃H₇, CH₃, C₂H₅ und C₃H₇; m = 0, 1, 2, 3, 4, 5, 6, 7 oder 8 ist.

3. Verfahren zur Herstellung einer Verbindung nach Anspruch 1 oder Anspruch 2, wobei die Verbindung aus Senfverbindungen durch Umsetzen mit Verbindungen mit der allgemeinen Formel der Struktur 3a unter Verwendung eines Kopplungsmittels hergestellt wird, das ausgewählt ist aus der Gruppe bestehend aus N,N',-Dicyclohexylcarbodiimid, N,N'-Diisopropylcarbodiimid, O-(Benzotriazol-1-yl)-N,N,N',N'-tetramethyluroniumtetrafluorborat, O-(Benzotriazol-1-yl)-N,N,N',N'-tetramethyluroniumhexafluorophosphat, Benzotriazol-1-yl-oxy-tris(dimethylamino)phosphoniumhexafluorophosphat 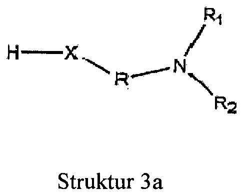
wobei R ausgewählt ist aus der Gruppe bestehend aus einem verzweigten oder geradkettigen -(CH₂)ₙ-und n = 0, 1, 2, 3, 4, 5, 6, 7, 8, 9 oder 10 ist;
R₁ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
R₂ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
X ausgewählt ist aus der Gruppe bestehend aus O, S und NH.

4. Verfahren zur Herstellung einer Verbindung nach Anspruch 1 oder Anspruch 2, wobei Metallsalze, Salze organischer Basen oder immobilisierte Basensalze von Senf-Verbindungen mit Verbindungen mit der allgemeinen Formel der Struktur 4a 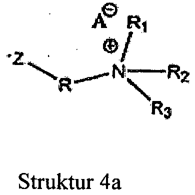 umgesetzt werden,
wobei R ausgewählt ist aus der Gruppe bestehend aus einem verzweigten oder geradkettigen -(CH₂)ₙ-und n = 0, 1, 2, 3, 4, 5, 6, 7, 8, 9 oder 10 ist;
R₁ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
R₂ ausgewählt ist aus der Gruppe bestehend aus H, Alkyl, Alkyloxy, Alkenyl und Alkinyl Resten mit bis zu 12 Kohlenstoffatomen, Aryl und Heteroaryl Resten;
R₃ H ist;
Z Halogen oder p-Toluolsulfonyl ist;
A⁻ ausgewählt ist aus beliebigen negativen Ionen.

5. Die Verbindung nach den Ansprüchen 1 und 2 oder eine Zusammensetzung, die mindestens eine Verbindung nach den Ansprüchen 1 und 2 als aktiven Bestandteil umfasst, zur Verwendung in einem Verfahren zur Behandlung von Mensch oder Tier, wobei der behandelte Zustand ausgewählt ist aus der Gruppe bestehend aus Hautkrebs, Brustkrebs, Mundkrebs, Dickdarm-Mastdarm-Krebs, Krebs der Atemwege, Genitalkrebs, Leukämie, anderen Krebsarten und Psoriasis, und die Verbindung oder Zusammensetzung transdermal oder oral verabreicht wird.

6. Transdermale therapeutische Anwendungssysteme, die eine Verbindung nach Anspruch 1 oder 2 oder eine Zusammensetzung umfassen, die mindestens eine Verbindung nach Anspruch 1 oder 2 als aktiven Bestandteil umfasst, zur Verwendung in einem Verfahren zur Behandlung von Mensch oder Tier, wobei der behandelte Zustand ausgewählt ist aus der Gruppe bestehend aus Hautkrebs, Brustkrebs, Mundkrebs, Dickdarm-Mastdarm-Krebs, Krebs der Atemwege, Genitalkrebs, Leukämie, anderen Krebsarten und Psoriasis.

7. Die transdermalen therapeutischen Applikationssysteme zur Verwendung nach Anspruch 6, **dadurch gekennzeichnet, dass** diese Systeme eine Bandage oder ein Pflaster, die/das eine wirkstoffhaltige Matrixschicht und eine undurchlässige Deckschicht umfasst, sind.

8. Die transdermalen therapeutischen Applikationssysteme zur Verwendung nach den Ansprüchen 6 oder 7, **gekennzeichnet durch** ein Wirkstoffreservoir, das einen durchlässigen Boden hat, der der Haut zugewandt ist.

9. Die transdermalen therapeutischen Anwendungssysteme zur Verwendung nach einem der Ansprüche 6-8, **gekennzeichnet durch** eine Steuereinrichtung zur Steuerung der Freisetzungsrate, so dass dieses System ständig optimale therapeutische Blutspiegel der Senf-Verbindungen erreicht, zur Steigerung der Effektivität und zur Reduktion der Nebenwirkungen der Senf-Verbindungen.

## Revendications

1. Composé choisi parmi le groupe comprenant le bromhydrate d'ester N,N-diéthylaminoéthylique de N,N-bis(2-chloroéthyl)aminophosphamide, le bromhydrate d'ester N,N-diéthylaminoéthylique de 4-[bis(2-chloroéthyl)amino]-N-acétyl-L-phénylalanine, le 4-[bis(2-méthylsulfonyléthyl)amino]benzènebutyrate de diéthylaminoéthyle · HBr, et un composé de formule générale de structure la : 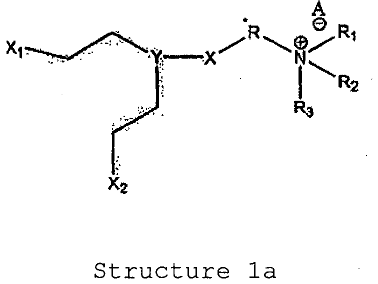
Où R₁ est choisi parmi le groupe comprenant H, les résidus alkyle, alkyloxy, alcényle et alcynyle possédant jusqu'à 12 atomes de carbone, et les résidus aryle et hétéroaryle ;
R₂ est choisi parmi le groupe comprenant H, les résidus alkyle, alkyloxy, alcényle et alcynyle possédant jusqu'à 12 atomes de carbone, et les résidus aryle et hétéroaryle ;
R₃ est H ;
A⁻ est choisi parmi n'importe quels ions négatifs ;
R est choisi parmi le groupe comprenant une chaîne ramifiée ou linéaire, -(CH₂)ₙ- où n = 0, 1, 2, 3, 4, 5, 6, 7, 8, 9 ou 10 ;
X est choisi parmi le groupe comprenant O, S et NH ;
X₁ est choisi parmi le groupe comprenant Cl, Br, F, I et OSO₂R₄ (R₄ est un groupe aryle ou hétéroaryle) ;
X₂ est choisi parmi le groupe comprenant Cl, Br, F, I et OSO₂R₄ (R₄ est un groupe aryle ou hétéroaryle) ;
Y est choisi parmi le groupe comprenant 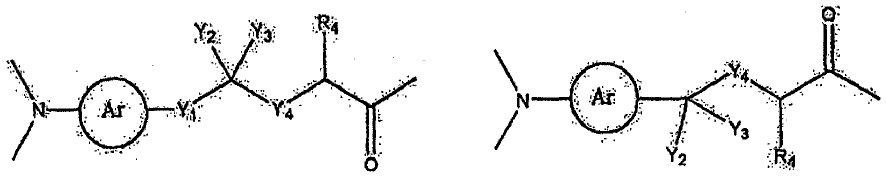 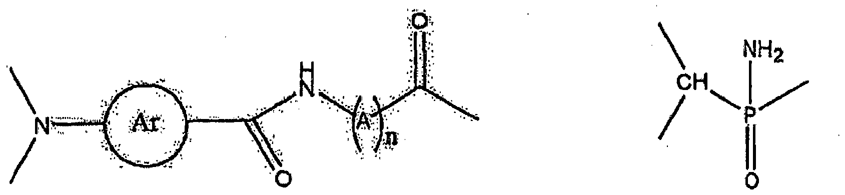
où Y₁ est choisi parmi le groupe comprenant CH₂, O, S, et NH ;
Y₂ et Y₃ sont choisis indépendamment dans le groupe comprenant H, OH, NHCOCH₃, NHCOC₂H₅, Cl, F, Br et I, ou bien, pris ensemble, sont l'oxygène ou 2 hydrogènes ;
Y₄ est choisi parmi le groupe comprenant CH₂, -(CH₂)ₚ- où p = 0, 1, 2, 3, 4, 5, 6 ou 7, O, S, et NH ;
R₄ est choisi parmi le groupe comprenant H, CH₂CONH₂, CH₂CH₂CONH₂, CH₂COOCH₃, CH₂CNOOC₂H₅, CH₂NHCOCH₃, CH₂CH₂NHCOCH₃, CH₂CH₂CH₂NHCOCH₃, CH₂CH₂CH₂CH₂NHCOCH₃, les résidus alkyle, alkyloxy, alcényle et alcynyle possédant jusqu'à 12 atomes de carbone, les résidus aryle et hétéroaryle ;
A est choisi parmi le groupe comprenant les résidus d'acide α-aminé et d'acide β-aminé ; 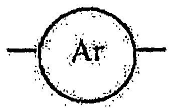 est choisi dans le groupe comprenant : 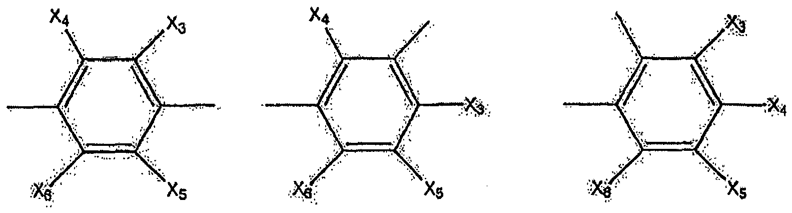 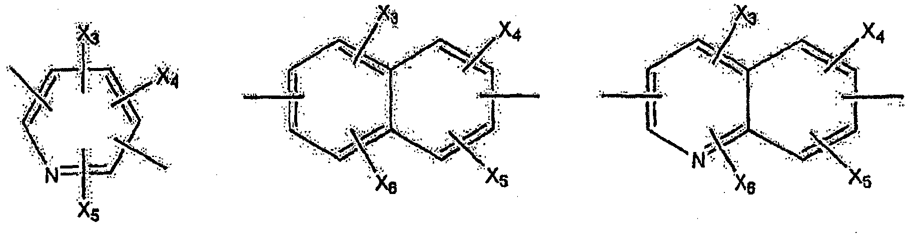 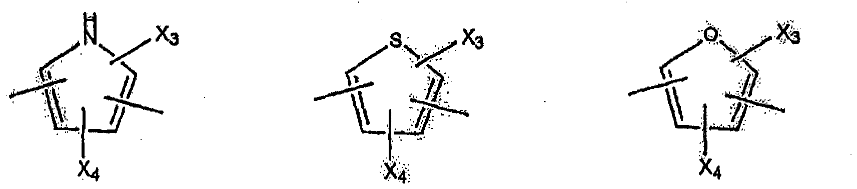
où X₃, X₄, X₅ et X₆ sont choisis indépendamment parmi le groupe comprenant H, F, Cl, Br, I, NHCOCH₃, NO₂, CN, CF₃, OCH₃, SCH₃, NH₂, NHCH₃, OCH3, OC₂H₅, OC₃H₇, CH₃, C₂H₅, et C₃H₇, où, lorsque les liaisons ne sont pas reliées à des atomes du cycle aryle ou hétéroaryle, cela signifie que les liaisons peuvent être placées à n'importe quelle endroit du cycle.

2. Composé ayant une formule générale correspondant à la structure 2a : 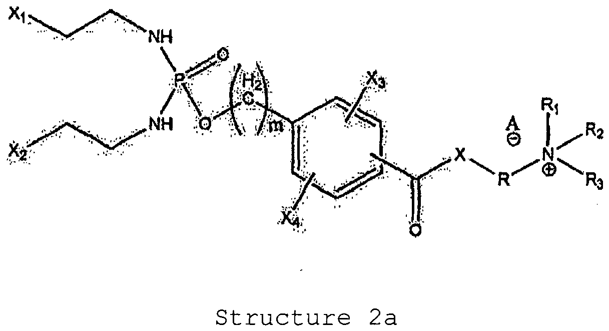
où R₁ est choisi parmi le groupe comprenant H, les résidus alkyle, alkyloxy, alcényle et alcynyle possédant jusqu'à 12 atomes de carbone, et les résidus aryle et hétéroaryle ;
R₂ est choisi parmi le groupe comprenant H, les résidus alkyle, alkyloxy, alcényle et alcynyle possédant jusqu'à 12 atomes de carbone, et les résidus aryle et hétéroaryle ;
R₃ est H ;
A⁻ est choisi parmi n'importe quels ions négatifs ;
R est choisi parmi le groupe comprenant une chaîne ramifiée ou linéaire, -(CH₂)ₙ- où n = 0, 1, 2, 3, 4, 5, 6, 7, 8, 9 ou 10, les résidus aryle et hétéroaryle ;
X est choisi parmi le groupe comprenant O, S et NH ;
X₁ est choisi parmi le groupe comprenant Cl, Br, F, I et OSO₂R₄ (R₄ est un groupe aryle ou hétéroaryle) ;
X₂ est choisi dans le groupe comprenant Cl, Br, F, I et OSO₂R₄ (R₄ est un groupe aryle ou hétéroaryle) ;
X₃ et X₄ sont choisis indépendamment parmi le groupe comprenant H, F, Cl, Br, I, NO₂, CN, CF₃, NHCOCH₃, OCH₃, SCH₃, NH₂, NHCH₃, OCOCH₃, OCOC₂H₅, OC₂H₅, OC₃H₇, CH₃, C₂H₅, et C₃H₇ ;
m = 0, 1, 2, 3, 4, 5, 6, 7 ou 8.

3. Procédé de préparation d'un composé selon la revendication 1 ou 2, dans lequel le composé est préparé à partir de composés de moutarde par réaction avec des composés de formule générale de structure 3a, en utilisant un réactif de couplage choisi parmi le groupe comprenant le N,N'-dicyclohexylcarbodiimide, le N,N'-diisopropylcarbodiimide, le tétrafluoroborate d'O-(benzotriazol-1-yl)-N,N,N',N'-tétraméthyluronium, l'hexafluorophosphate d'O-(benzotriazol-1-yl)-N,N,N',N'-tétraméthyluronium, l'hexafluorophosphate de benzotriazol-1-yloxytris(diméthylamino)phosphonium, 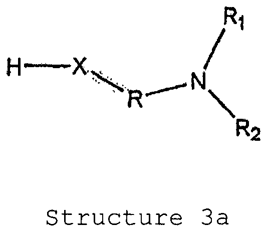
où R est choisi parmi le groupe comprenant une chaîne ramifiée ou linéaire, -(CH₂)ₙ- où n = 0, 1, 2, 3, 4, 5, 6, 7, 8, 9 ou 10 ;
R₁ est choisi parmi le groupe comprenant H, les résidus alkyle, alkyloxy, alcényle et alcynyle possédant jusqu'à 12 atomes de carbone, et les résidus aryle et hétéroaryle ;
R₂ est choisi parmi le groupe comprenant H, les résidus alkyle, alkyloxy, alcényle et alcynyle possédant jusqu'à 12 atomes de carbone, et les résidus aryle et hétéroaryle ;
X est choisi parmi le groupe comprenant O, S et NH.

4. Procédé de préparation d'un composé selon la revendication 1 ou 2, dans lequel des sels métalliques, des sels de bases organiques, ou des sels de bases immobilisés de composés de moutarde sont mis à réagir avec des composés de formule générale de structure 4a 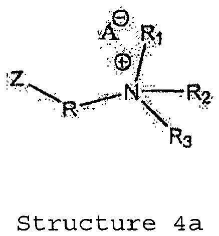
où R est choisi parmi le groupe comprenant une chaîne ramifiée ou linéaire, -(CH₂)ₙ- et n = 0, 1, 2, 3, 4, 5, 6, 7, 8, 9 ou 10 ;
R₁ est choisi parmi le groupe comprenant H, les résidus alkyle, alkyloxy, alcényle et alcynyle possédant jusqu'à 12 atomes de carbone, et les résidus aryle et hétéroaryle ;
R₂ est choisi parmi le groupe comprenant H, les résidus alkyle, alkyloxy, alcényle et alcynyle possédant jusqu'à 12 atomes de carbone, et les résidus aryle et hétéroaryle ;
R₃ est H ;
Z est un halogène ou p-toluènesulfonyle ;
A⁻ est choisi parmi n'importe quels ions négatifs.

5. Composé selon les revendications 1 et 2 ou composition comprenant au moins un composé selon les revendications 1 et 2 en tant que principe actif, destiné à être utilisé dans une méthode thérapeutique chez l'Homme ou l'animal, dans lequel l'état traité est choisi parmi le groupe comprenant le cancer de la peau, le cancer du sein, le cancer de la bouche, le cancer colorectal, le cancer de l'appareil respiratoire, le cancer génital, la leucémie, d'autres cancers et le psoriasis, le composé ou la composition est administré par voie transdermique ou orale.

6. Systèmes thérapeutiques d'application par voie transdermique comprenant un composé selon la revendication 1 ou 2 ou une composition comprenant au moins un composé selon la revendication 1 ou 2 en tant que principe actif, destinés à être utilisés dans une méthode thérapeutique chez l'Homme ou l'animal, dans lesquels l'état traité est choisi parmi le groupe comprenant le cancer de la peau, le cancer du sein, le cancer de la bouche, le cancer colorectal, le cancer de l'appareil respiratoire, le cancer génital, la leucémie, d'autres cancers et le psoriasis.

7. Systèmes thérapeutiques d'application par voie transdermique destinés à être utilisés selon la revendication 6, **caractérisés en ce que** ces systèmes sont un pansement ou un patch comprenant une couche de matrice contenant une substance active et une couche de support imperméable.

8. Systèmes thérapeutiques d'application par voie transdermique destinés à être utilisés selon la revendication 6 ou 7, **caractérisés en ce qu'**ils ont un réservoir de substance active, qui présente un fond perméable faisant face à la peau.

9. Systèmes thérapeutiques d'application par voie transdermique destinés à être utilisés selon l'une quelconque des revendications 6 à 8, **caractérisés par** un moyen de contrôle pour contrôler la vitesse de libération, en permettant à un tel système d'atteindre des concentrations sanguines thérapeutiques constamment optimales des composés de moutarde pour améliorer l'efficacité et diminuer les effets secondaires des composés de moutarde.
